## EUROPEAN PATENT SPECIFICATION

(12)

(45) Date of publication of patent specification: **10.03.93** (51) Int. Cl.⁵: **G01N 33/543**, G01N 33/563

(21) Application number: **87108681.5**

(22) Date of filing: **16.06.87**

The file contains technical information submitted after the application was filed and not included in this specification

(54) **Kit for use in sandwich immunoassay.**

(30) Priority: **19.06.86 JP 141375/86**
**23.06.86 JP 144973/86**
**03.04.87 JP 82512/87**
**16.06.87 JP 149325/87**

(43) Date of publication of application:
**23.12.87 Bulletin 87/52**

(45) Publication of the grant of the patent:
**10.03.93 Bulletin 93/10**

(84) Designated Contracting States:
**DE FR GB**

(56) References cited:
**EP-A- 0 125 893**
**WO-A-85/02258**
**US-A- 4 414 324**

**JOURNAL OF IMMUNOLOGICAL METHODS, vol. 83, no. 2, 1985, Elsevier Science Publishers B.V., Amsterdam, NL; H.TANIMORI et al.: "A new solid support for sandwich enzyme immunoassays of human immunoglobulin G" pages 327-336**

(73) Proprietor: **Kabushiki Kaisha Meidensha**
**1-17, Ohsaki 2-chome**
**Shinagawa-ku Tokyo 141(JP)**

(72) Inventor: **Aoyagi, Shigeo**
**11-7, Sugamachi**
**Shinjuku-ku Tokyo(JP)**
Inventor: **Kusumi, Miyoko**
**13-13, Nakamachi 2-chome**
**Koganei-shi Tokyo(JP)**
Inventor: **Matsuyuki, Akira**
**12-2-805, Yokokawa 4-chome**
**Sumida-ku Tokyo(JP)**
Inventor: **Oshima, Nobuo**
**32-9, Higashiminemachi**
**Ota-ku Tokyo(JP)**

(74) Representative: **Dipl.-Phys.Dr. Manitz Dipl.-Ing., Dipl.-W.-Ing. Finsterwald Dipl.-Ing. Grämkow Dipl.-Chem.Dr. Heyn Dipl.-Phys. Rotermund Morgan, B.Sc.(Phys.) Robert-Koch-Strasse 1 W-8000 München 22 (DE)**

EP 0 249 955 B1

CHEMICAL ABSTRACTS, vol. 90, no. 3, 05 January 1979, Columbus, Ohio, US; H.G.KOPP et al.: "Problems connected with the production of highly specific antisera against prostaglandin E2 (PGE2) and pros- taglandin A2 (PGA2) for radioimmunoassay" p. 270, abstract no. 18602r

**Description**

BACKGROUND OF THE INVENTION

This invention relates to sandwich immunoassays utilizing immunoreaction for measuring an extremely small quantity of an ingredient included in blood or other organic samples and, more particularly, to an improved kit for use in such sandwich immunoassays to increase the accuracy of measurement of the ingredient.

Immunoassays have been used widely for measuring an extremely small quantity of an ingredient included in blood or other organic samples by utilizing immunoreaction or antigen-antibody reaction. Such immunoassays are generally of the two types; the "competitive immunoassay" and the "non-competitive immunoassay". The competitive immunoassay employs a predetermined quantity of labeled antigen which binds to a predetermined quantity of antibody in a competitive manner with target antigen. The quantity of the target antigen is measured from the quantity of the labeled antigen binding to the antibody or the quantity of the labeled antigen which does not bind to the antibody. In the competitive immunoassay, however, the labeled antigen is required to have a high concentration for its reaction to the antibody. In addition, the competitive immunoassay exhibits a poor measurement sensitivity as low as about a twentieth of the quantity of the labeled antigen.

The non-competitive immunoassay is represented by a sandwich immunoassay which employs a solid phase coated on its surface with an excessive quantity of insolubilized antibody to trap a target antigen to be measured. The solid phase is washed and then incubated with an excessive amount of labeled antibody so that the labeled antibody can react with the trapped target antigen. The excessive labeled antibody is washed out away from the solid phase. The quantity of the target antigen is measured from the quantity of the label provided on the labeled antibody binding to the antigen. Although the sandwich immunoassay can provide a high measurement sensitivity, there is a tendency of non-specific binding of the labeled antibody to the solid phase, causing a considerable reduction in the sensitivity and measurable range of the sandwich immunoassay.

A sandwich enzyme immunoassay employing solid-phase and labeled antibodies originated from guinea pig is disclosed in J. Immunol. Methods 83/2, 1985, 327 - 336.

EP-A-O 125 893 describes a method for the quantitative analysis of antigen by an enzyme-antibody bridge method. In one step of the multi-step method a second antibody in a solution which can contain normal animal serum either is added to an insoluble carrier bound to a first antibody and to an antigen or is added simultaneously with the antigen to the insoluble carrier bound to the first antibody.

US-A-4 414 324 concerns an immunoassay sample determination process wherein one of the components of an antigen-antibody reaction is insolubilized on the walls of a vessel, the other component is labeled and the sample being determined is added together with the labeled component to the vessel. The labeled component binds in a competitive manner with the sample to the insolubilized component. For minimizing non-specific interactions a site-deactivating medium, e.g. an animal-derived serum, is covalently bound to the walls of the vessel and then the insolubilized component is covalently attached to said medium.

SUMMARY OF THE INVENTION

It is a main object of the invention to provide an improved kit for use in a sandwich immunoassay to reduce the degree of non-specific binding of the labeled antibody to the solid phase to a considerable extent.

It is still another object of the invention to provide an improved kit for use in a sandwich immunoassay to increase the sensitivity and sensitive range of the sandwich immunoassay.

It is still another object of the invention to provide an improved kit for use in a sandwich immunoassay to reduce the degree of deviation of the sandwich immunoassay.

According to the present invention there is provided a kit for use in a sandwich immunoassay employing labeled and solid-phase antibodies reactive with an antigen in the presence of a reaction solution to bind the antigen between the labeled and solid-phase antibodies, said kit containing a solid-phase antibody having the antibody bound to a solid-phase, a labeled antibody having a label enzyme bound to the antibody at least one of the labeled and solid-phase antibodies being originated from guinea pig, and a reaction solution containing normal guinea pig serum for reducing the degree of non-specific binding of the labeled antibody to the solid phase.

BRIEF DESCRIPTION OF THE DRAWINGS

This invention will be described in greater detail by reference to the following description taken in connection with the accompanying drawings, in which:

Fig. 1 is a graph showing the relationship between chemiluminescent intensity and hCG quantity;

Fig. 2 is a graph showing the relationship between chemiluminescent intensity and AFP quantity;

Fig. 3 is a schematic diagram showing the IgG structure;

Fig. 4 is a graph plotting non-specific binding percentage with respect to given ratios of normal guinea pig serum to reaction solution A;

Fig. 5 is a graph showing between chemiluminescent intensity and hCG antity;

Fig. 6 is a graph plotting non-specific binding percentage with respect to given examples; and

Fig. 7 is a graph showing the relationship between chemiluminescent intensity and hCG quantity.

DETAILED DESCRIPTION OF THE INVENTION

One embodiment of the invention will be described. A target antigen is assayed by a sandwich enzyme immunoassay (EIA) technique employing first and second antibodies reactive in a specific manner with the target antigen in the presence of a reaction solution to trap the target antigen between the first and second antibodies. The antibodies are obtained from serum drawn from a selected animal into which the target antigen is injected. The first antibody is referred to as a solid-phase antibody which is coated on a solid phase such is polystyrene ball. The second antibody is referred to as a labeled antibody which has a label enzyme bound thereon. The label enzyme may be glucose oxidase. peroxidase, alkaline phosphatase, $\beta$-D-galactosidase, or the like. A chemiluminescence technique is used to measure the quantity of the label enzyme which corresponds to the quantity of the target antigen. These procedures will be decribed in greater detail on an assumption that the target antigen is human chorionic gonadotropin (hCG), the selected animal is guinea pig, and the label enzyme is glucose oxidase (GOD). The reaction solution used is 0.056 mol/l sodium phosphate buffer (pH 6.3) containing 0.1% sodium aside, 0.2% bovine serum albumin (BSA) and 0.337% NaCl. This reaction solution is referred to as reaction solution A.

First of all. 3000 IU (international unit) of hCG (antigen) is injected six times under the back skin of a guinea pig at intervals of one week. On the seventh week, 1000 IU of booster is injected into the guinea pig. After five days, a proper amount of blood is drawn from the guinea pig.

Guinea pig anti-hCG immunoglobulin G (IgG) is purified from the drawn serum in the following manner. First of all. 2 ml saturated ammonium sulfate is dripped into 2 ml of serum. The resulting solution is agitated slowly at a temperature of 4°C for two or three hours. The resulting cloudy solution is then placed in a centrifugation tube. Centrifugation is performed at a temperature of 4°C with a contrifugal separator rotating at a speed of 3000 rpm for 15 minutes. The supernatuant is removed from the centrifugation tube, whereas the precipitate is dissolved with 2 ml of 0.1 mol/l sodium phosphate buffer (pH 6.0) containing 5 mmol/l ethylenediaminetetraacetic acid (EDTA). The resulting solution is eluted at a flow rate of 6 ml/hour on Sephacryl S-300 (a trademark of Pharmacia Fine Chemicals, Sweden) column (1 x 90 cm) equilibrated with 0.1 mol/l sodium phosphate buffer (pH 6.0) containing 5 mmol/l ethylenediaminetetraacetic acid (EDTA). The eluted solution is divided into 1 ml of samples. The guinea pig anti-hCG IgG fractions, which absorb light at 280 nm, are gathered from the respective samples.

The resulting guinea pig anti-hCG IgG is coated through physical absorption on a solid phase by soaking a polystyrene ball (6.5 mm in diameter) available from Ichiko Co., Japan at a temperature of 4°C in 0.1 mol/l sodium phosphate buffer (pH 7.5) containing 0.1 mg/ml guinea pig anti-hCG IgG all the night through. The anti-hOC Igc-coated polystyrene ball is washed three times in 0.1 mol/l sodium phosphate buffer (pH 7.5) and then washed three times with 0.01 mol/l sodium phosphate buffer (pH 7.0) containing 0.1 mol/l NaCl, 0.1% bovine serum albumin (BSA) and 0.1% $NaN_3$. The washed polystyrene ball is stored at a temperature of 4°C in 0.01 mol/l sodium phosphate buffer (pH 7.0) containing 0.1 mol/l NaCl, 0.1% bovine serum albumin (BSA) and 0.1% $NaN_3$.

The labeled antibody, guinea pig anti-hCG IgG-GOD is prepared through the following three steps:

(1) Maleimide glucose oxidase (Maleimide GOD) is prepared as follows. Succinimidyl-4-maleimidobutyrate (GMB S) is added to about 3 mg of glucose oxidase (GOD) placed in each of four test tubes containing 0.3 ml of 0.1 mol/l sodium phosphate buffer (pH 7.0) to produce a solution having a GOD/GMB S ratio of 1/50. This solution is incubated at a temperature of 30°C for one hour. The resulting solution is desalted at a flow rate of 12 ml/hour on a Sephadex G-25 (a trademark of Pharmacia Fine Chemicals, Sweden) column (1 x 30 cm) equilibrated with 0.1 mol/l sodium phosphate buffer (pH 6.0). The desalted solution is divided into 1 ml of maleimide GOD samples.

EP 0 249 955 B1

(2) The sulfhydrated guinea pig anti-hCG immunoglobulin G (SH-IgG) is prepared as follows. First of all, 2 ml of IgG solution containing 10 mg of guinea pig anti-hCG IgG, 0.1 mol/l sodium phosphate buffer (pH 6.0) and 5 mmol/l ethylenediaminetetraacetic acid (EDTA) is prepared. The IgG solution is added to a solution containing S-acetylmercaptosuccimic anhydride (ASMSa) dissolved in N, N-dimethylformamide (DMF) to Produce a solution having an IgG/ASMSa ratio, by mol concentration, of 1/300. The resulting solution is agitated at a temperature of 30°C for 30 minutes. In succession, this solution is added with 0.1 ml of 0.1 mol/l Tris-HCℓ buffer (pH 7.0). 0.02 ml of 0.1 mol/l ethylenediaminetetraacetic acid (EDTA) (pH 7.0) and 0.1 ml of 1 mol/l hydroxylamine hydrochloride (pH 7.0). The resulting solution is incubated at a temperature of 30°C for 4 minutes and then the resulting solution is desalted at a flow rate of 12 ml/hour on a Sephadex G-25 (a trademark of Pharmacia Fine Chemicals, Sweden) column (1x30 cm) equillbrated with 0.1 mol/l sodium phosphate buffer (pH 6.0) containing 5 mmol/l ethylenediaminetetraacetic acid (EDTA). The desalted solution is divided into 1 ml of SH-IgG samples. These SH-IgG samples are concentrated in an ice-cooled collodion bag.

(3) The labeled antibody was prepared as follows. The maleimide GOD is added to the SH-IgG solution with the same mol concentration as the maleimide GOD. After the resulting solution is held one hour at a temperature of 30°C and then held at a temperature of 4°C stationary all the night through, it is eluted at a flow rate of 6 ml/hour on a Sephacryl S-300 (a trademark of Pharmacia Fine Chemicals, Sweden) column (1 x 90 cm) equilibrated with 0.1 mol/l sodium phosphate buffer solution (pH 6.5) containing 5 mmol/l ethylenediaminetetraacetic acid (EDTA). The eluted solution is divided into 1 ml of samples. The guinea pig anti-hCG IgG-GOD (labeled antibody) is gathered from the respective samples and stored at a temperature of 4°C in a solution containing 0.1% $NaN_3$ and 0.1% bovine serum albumin (BSA).

The labeled antibody (guinea pig anti-hCG IgG-GOD) is bound through the antigen (hCG) to the solid-phase antibody (guinea pig anti-hCG IgG) in the presence of the reaction solution A (0.056 mol/l sodium phosphate buffer (pH 6.3) containing 0.1% sodium azide. 0.2% bovine serum albumin (BSA) and 0.337% NaCl) in the following manner. The anti-hCG IgG-coated polystyrene ball is incubated at room temperature with hCG (antigen) diluted with the reaction solution A. The polystylene ball is then washed three times with distilled water. The washed polystyrene ball is incubated at room temperature with anti-hCG IgG-GOD (labeled antibody) in the reaction solution A. The polystyrene ball is then washed again with distilled water and transferred into another clean assay tube.

A chemiluminescent technique is used to determine the quantity of the label enzyme; that is, the quantity of the antigen. First of all. 0.3 ml of 0.01 mol/l acetate buffer (pH 5.1) containing 0.5 mol/l glucose is added to the assay tube. The assay tube is held stationary at a temperature of 37°C for two hours. The resulting solution is taken to prepare a 0.1 ml sample. The sample is added with 0.5 ml of 0.2 mol/l carbonate buffer (pH 9.8) containing $2 \times 10^{-7}$ mol/l luminol and then 0.5 ml of $6 \times 10^{-3}$ mol/l potassium ferricyanide. The concentration of the resulting hydrogen peroxide is measured from the light emitted upon chemiluminescent reaction of the luminol and potassium ferricyanide and the hydrogen peroxide by a Luminometer UPD-8000 (a trademark of Meidensha Electric Mfg., Co., Japan) which starts its operation 15 second after the luminol and potassium ferricyanide are added to the assay tube and counts the light emitted for 30 seconds.

Although the antigen has been described in connection with human chorionic gonadotropin (hCG), it is to be noted that it may be human chorionic gonadotropin $\beta$ (hCG$\beta$), $\alpha$-fetoprotein (AFP), anti-carcinoembryonic antigen (CEA) or the like. Anti-AFp IgG is purified from serum drawn from selected one of animals including guinea pig, rabbit, goat and the like into which AFP is injected six times at intervals of one week and a booster is injected on the seventh week. Anti-CEA IgG is purified from serum drawn from selected one of animals including guinea pig, rabbit, goat and the like into which CEA is injected six times at intervals of one week and a booster is injected on the seventh week,

A serious problem involved with such sandwich enzyme immunoassay (EIA) is the tendency of non-specific binding of a relatively great quantity of labeled antibody to the solid phased degrading the accuracy of sanwich enzyme immunoassay. The inventors found that the degree of non-specific binding of the labeled antibody to the solid phase was reduced when at least one of the solid-phase and labeled antibodies was prepared from blood serum originated from guinea pig. A large number of tests were conducted to prove the effective combinations of the solid-phase and labeled antibodies.

The degree of non-specific binding of the labeled antibody to the solid phase was determined. For this purpose, an IgG-coated polystyrene ball was incubated at room temperature with IgG-GOD diluted hundredfold with 0.3 ml of reaction solution A in an assay tube all the night through. The polystyrene ball was washed three times with the reaction solution A and then transferred into another clean assay tube. Following this, 0.3 ml of 0.01 mol/l acetate buffer (pH 5.1) containing 0.5 mol/l glucose was added to the assay tube. The assay tube was held stationary at a temperature of 37°C far two hours. The resulting

5

solution was taken to prepare a 0.1 ml sample. The sample was added with 0.5 ml of 0.2 mol/l carbonate buffer (pH 9.8) containing $2 \times 10^{-7}$ mol/l luminol and then with 0.5 ml of $6 \times 10^{-3}$ mol/l potassium ferricyanide. The light emitted upon chemiluminescent reaction of the luminol and potassium ferricyanide and the resulting hydrogen peroxide was measured by a Luminometer UPD-8000 which started its operation 15 seconds after the luminol and pltassium ferricyanide were added to the assay tube and counted the light emitted for 30 seconds. The degree of non-specific binding of the labeled antibody to the solid phase was represented by the non-specific bonding ratio given as $C1/C2 \times 100$ (%) where C1 is the number of counts measured for the labeled antibody binding to the polystyrene ball and C2 is the number of counts corresponding to the whole amount of the labeled antibody used in the assay tube.

In one series of these tests, the solid-phase antibody used was originated from guinea pig and the labeled antibody used was originated from guinea pig.

Example 1 - The labeled antibody used was normal guinea pig IgG-GOD and the solid-phase antibody used was normal guinea pig IgG. The normal guinea pig IgG was Miles Co,, Lot No. 29. A normal Guinea pig IgG-coated polystyrene ball was incubated with the normal guinea pig IgG-GOD in the presence of the reaction solution A mixed with guinea pig serum.

Example 2 - The labeled antibody used was guinea pig anti-hCG IgG-GOD and the solid-phase antibody used was guinea pig anti-hCG IgG. A guinea pig anti-hCG IgG-coated polystyrene ball was incubated with the guinea pig anti-hCG IgG-GOD in the presence of the reaction solution A mixed with guinea pig serum.

Example 3 - The labeled antibody used was guinea pig anti-AFP IgG-GOD and the solid-phase antibody used was guinea pig anti-AFP IgG. A guinea pig anti-AFP IgG-coated polystyrene ball was incubated with the guinea pig anti-AFP IgG-GOD in the presence of a reaction solution.

Example 4 - The labeled antibody used was guinea pig anti-CEA IgG-GOD and the solid-phase antibody used was guinea pig anti-CEA IgG. A guinea pig anti-CEA IgG-coated polystyrene ball was incubated with the guinea pig anti-CEA IgG-GOD in the presence of the reaction solution A mixed with guinea pig serum.

Example 5 - The labeled antibody used was guinea pig anti-insulin IgG-GOD and the solid-phase antibody was guinea pig anti-insulin IgG. The guinea pig anti-insulin IgG was Milea Co., Lot No. 23. A guinea pig anti-insulin IgG-coated polystyrene ball was incubated with the guinea pig anti-insulin IgG-GOD in the presence of the reaction solution A mixed with guinea pig serum.

For comparison of the non-specific binding reduction effect obtainable by the invention, tests were conducted for the following comparative examples where neither the solid-phase antibody nor the labeled antibody was originated from guinea pig.

Comparative Example 1 The labeled antibody used was normal rabbit IgG-GOD and the solid-phase antibody used was normal rabbit IgG. The normal rabbit IgG was Miles Co., Lot No. 0019. A normal rabbit IgG-coated polystyrene ball was incubated with the normal rabbit IgG-GOD in the presence of the reaction solution A mixed with guinea pig serum.

Comparative Example 2 - The labeled antibody used was rabbit anti-hCG IgG-GOD and the solid-phase antibody used was rabbit anti-hCG IgG. The rabbit anti-hCG IgG was EY Co., Lot No. 021304. A rabbit anti-hCG IgG-coated polystyrene ball was incubated with the rabbit anti-hCG IgG-GOD in the presence of the reaction solution A mixed with guinea pig serum.

Comparative Example 3 - The labeled antibody used was rabbit anti-AFP IgG-GOD and the solid-phase antibody used was rabbit anti-AFP IgG. The rabbit anti-AFP IgG was Nordic Co., Lot No. 16-184. A rabbit anti-AFP IgG-coated polystyrene ball was incubated with the rabbit anti-AFP IgG-GOD in the presence of the reaction solution A mixed with guinea pig serum.

Comparative Example 4 - The labeled antibody used was rabbit anti-CEA IgG-GOD and the solid-phase antibody used was rabbit anti-CEA IgG. The rabbit anti-CEA IgG was Zymed Co.. Lot No. 40925. A rabbit anti-CEA IgG-coated polystyrene ball was incubated with the rabbit anti-CEA IgG-GOD in the presence of the reaction solution A mixed with guinea pig serum.

In another series of these tests, the solid-phase antibody used was originated from guinea pig and the labeled antibody used was originated from rabbit or goat.

Example 6 - The labeled antibody used was rabbit anti-hCG IgG-GOD and the solid-phase antibody used was guinea pig anti-hCG IgG. The rabbit anti-hCG IgG was EY Co., Lot No. 021304. A guinea pig anti-hCG IgG-coated polystyrene ball was incubated with the rabbit anti-hCG IgG-GOD in the presence of the reaction solution A mixed with guinea pig serum.

Example 7 - The labeled antibody used was rabbit anti-AFP IgG-GOD and the solid-phase antibody used was guinea pig anti-AFP IgG. The rabbit anti-AFP IgG was Nordic Co., Lot No. 16-184. A guinea pig anti-AFP IgG-coated polystyrene ball was incubated with the rabbit anti-AFP IgG-GOD in the presence of

the reaction solution A mixed with guinea pig serum.

Example 8 - The labeled antibody used was goat anti-AFP IgG-GOD and the solid-phase antibody used was guinea pig anti-AFP IgG. The goat anti-AFP IgG was Cappel Co., Lot No. 20176. A guinea pig anti-AFP IgG-coated polystyrene ball was incubated with the goat anti-AFP IgG-GOD in the presence of the reaction solution A mixed with guinea pig serum.

Example 9 - The labeled antibody used was goat anti-hCG IgG-GOD and the solid-phase antibody used was guinea pig anti-hCG IgG. A guinea pig anti-hCG IgG-coated polystyrene ball was incubated with the goat anti-hCG IgG-GOD in the present of the reaction solution A mixed with guinea pig serum.

In still another series of these tests, the solid-phase antibody used was originated from rabbit or goat and the labeled antibody used was originated from guinea pig.

Example 10 - The labeled antibody used was guinea pig anti-hCG IgG-GOD and the solid-phase antibody used was rabbit anti-hCG IgG. The rabbit anti-hCG IgG was EY Co., Lot No. 021304. A rabbit anti-hCG IgG-coated polystyrene ball was incubated with the guinea pig anti-hCG IgG-GOD in the presence of the reaction solution A mixed with guinea pig serum.

Example 11 - The labeled antibody used was guinea pig anti-AFP IgG-GOD and the solid-phase antibody used was rabbit anti-AFP IgG. The rabbit anti-AFP IgG was Nordic Co., Lot No. 16-134. A rabbit anti-AFP IgG-coated polystyrene ball was incubated with the guinea pig anti-AFP IgG-GOD in the presence of the reaction solution A mixed with guinea pig serum.

Example 12 - The labeled antibody used was guinea pig anti-APP IgG-GOD and the solid-phase antibody used was goat anti-AFP IgG. The goat anti-AFP IgG was Cappel Co., Lot No. 20176. A goat anti-APP IgG-coated polystyrene ball was incubated with the guinea pig anti-AFP IgG-GOD in the presence of the reaction solution A mixed with guinea pig serum.

For comparison of the non-specific binding reduction effect obtainable by the invention, tests were conducted for the following comparative examples where neither the solid-phase antibody nor the labeled antibody was originated from guinea pig.

Comparative Example 5 - The labeled antibody used was rabbit anti-hCG IgG-GOD and the solid-phase antibody used was rabbit anti-hCG IgG. A rabbit anti-hCG IgG-coated polystyrene ball was incubated with the rabbit anti-hCG IgG-GOD in the presence of the reaction solution A mixed with guinea pig serum.

Comparative Example 6 - The labeled antibody used was rabbit anti-AFP IgG-GOD and the solid-phase antibody used was rabbit anti-AFP IgG. A rabbit anti-AFP IgG-coated Polystyrene ball was incubated with the rabbit anti-AFP IgG-GOD in the presence of the reaction solution A mixed with guinea pig serum.

Comparative Example 7 - The labeled antibody used was goat anti-AFP IgG-GOD and the solid-phase antibody used was goat anti-AFP IgG. A goat anti-AFP IgG-coated polystyrene ball was incubated with the goat anti-AFP IgG-GOD in the presence of the reaction solution A mixed with guinea pig serum.

The results of the non-specific binding tests are illustrated in Table 1.

TABLE 1

| Example No. | Average Non-Specific Binding Percentage (%) | comparative Example No. | Average Non-Specific Binding Percentage (%) |
|---|---|---|---|
| 1 | 0.0089 | 1 | 0.148 |
| 2 | 0.0093 | 2 | 0.161 |
| 3 | 0.016 | 3 | 0.190 |
| 4 | 0.0094 | 4 | 0.094 |
| 5 | 0.014 | | |
| 6 | 0.020 | | |
| 7 | 0.023 | | |
| 8 | 0.019 | | |
| 9 | 0.018 | | |
| 10 | 0.020 | 5 | 0.161 |
| 11 | 0.016 | 6 | 0.190 |
| 12 | 0.018 | 7 | 0.078 |

The non-specific binding percentage ranges from 0.078% to 0.190% for combinations where neither the solid-phase antibody nor the labeled antibody is originated from guinea pig. The non-specific binding percentage range from 0.0089% to 0.023% for combinations where at least one of the solid-phase antibody and the labeled antibody is originated from guinea pig. It can be seen from the test results that the degree of non-specific binding of the labeled antibody to the solid phase can be reduced to a remarkable extent when at least one of the solid-phase antibody and the labeled antibody is originated from guinea pig.

The sensitivity and measurable range of such sandwich enzyme immunoassay was determined. For this purpose, an anti-hCG IgG-coated polystyrene ball was incubated at room temperature with 0.1 ml of hCG standard solution diluted with 0.2 ml of reaction solution A in an assay tube for six hours. The polystyrene ball was washed three times with the reaction solution A. The washed polystyrene ball was incubated with 0.1 ml of anti-hCG IgG-GOD labelled antibody diluted at a proper ratio with the reaction solution A. The assay tube was held stationary at room teperature all the night through. The polystyrene ball was then washed three times with the reaction solution A and transferred into another clean assay tube. Following this, 0.3 ml of 0.01 mol/l acetate buffer (pH 5.1) containing 0.5 mol/l glucose was added to the assay tube. The assay tube was held stationary at a temperature of 37°C for two hours. 0.1 ml of the reaction solution was pipetted into another assay tube. The tube was added with 0.5 ml of 0.2 mol/l carbonate buffer (pH 9.8) containing $2 \times 10^{-7}$ mol/l luminol and then with 0.5 ml of $6 \times 10^{-3}$ mol/l potassium ferricyanide. The light emitted upon chemiluminescent reaction of the luminol and the resulting hydrogen peroxide was measured by a Luminometer UPD-800 which started its operation 15 seconds after the luminol and potassium ferricyanide were added to the assay tube and counted the light emitted for 30 seconds. Similar procedures are repeated for anti-AFP IgG antibodies and anti-CEA IgG antibodies.

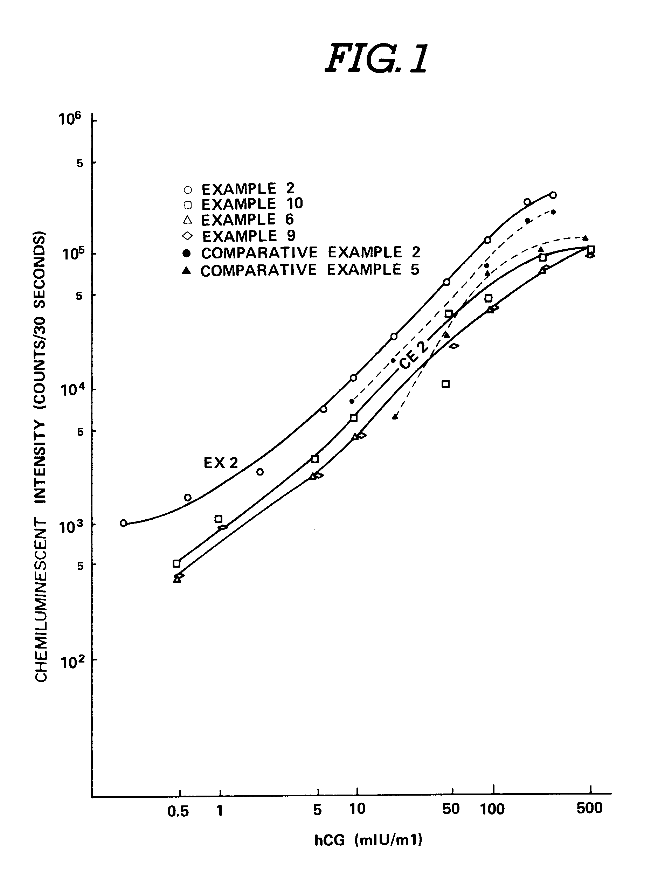

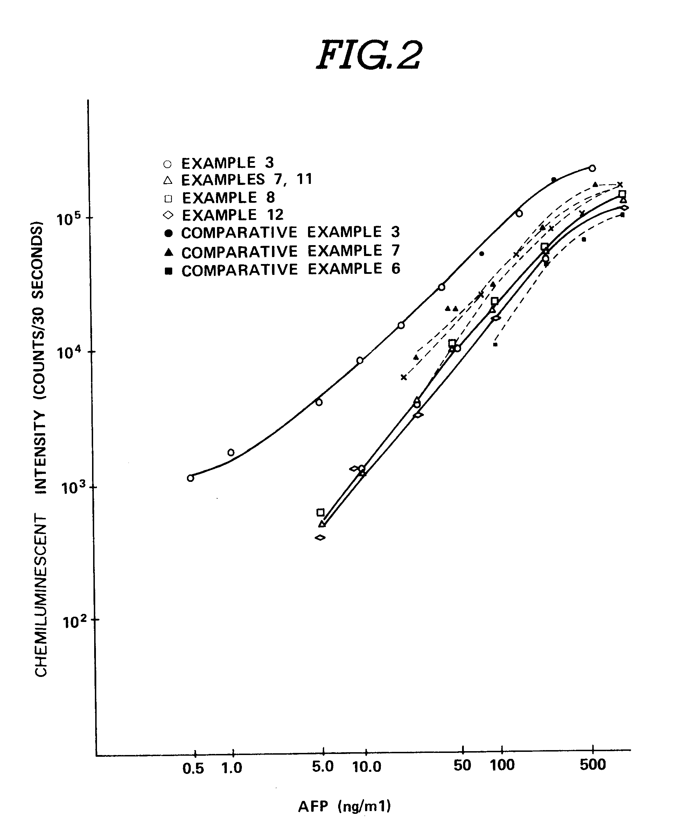

The results of the sandwich enzyme immunoassay sensitivity and measurable range are illustrated in Figs. 1 and 2. Fig. 1 relates to Examples 2, 6, 9 and 10 and Comparative Examples 2 and 5 where the antigen used was hCG. Fig. 2 relates to Examples 3, 7. 8. 11 and 12 and Comparative Examples 3, 6 and 7 where the antigen used was AFP. It can be seen from these test results that the sensitivity and measurable range of the sandwich enzyme immunoassay are superior when at least one of the solid-phase antibody and the labeled antibody is originated from guinea pig to those obtained when neither the solid-phase antibody nor the labeled antibody is originated from guinea pig.

Sandwich enzyme immunoassay was repeated ten times within a day (within assay) using the antibodies in Examples 2, 6, 9 and 10 and Comparative Examples 2 and 5. The results are shown in Tables 2 and 3. Similarly, sandwich enzyme immunoassay was repeated six times on different six days (between assay) using the antibodies in Examples 2, 6, 9 and 10 and comparative Examples 2 and 5. The results are shown in Tables 4 and 5.

TABLE 2

| Example No. | Number of Assay Repetitions | hCG (mIU/ml) Average ± Standard Value Deviation | | | Variation Coefficient (%) |
|---|---|---|---|---|---|
| 2 | 10 | 22 | ± | 1.7 | 7.7 |
| 2 | 10 | 110 | ± | 5.8 | 5.3 |
| 2 | 10 | 230 | ± | 15.1 | 6.6 |
| 6 | 10 | 23 | ± | 1.5 | 6.5 |
| 6 | 10 | 106 | ± | 8.8 | 8.3 |
| 6 | 10 | 224 | ± | 11.2 | 5.0 |
| 9 | 10 | 22 | ± | 1.7 | 7.7 |
| 9 | 10 | 110 | ± | 5.8 | 5.3 |
| 9 | 10 | 230 | ± | 15.1 | 6.6 |
| 10 | 10 | 21 | ± | 1.5 | 7.3 |
| 10 | 10 | 101 | ± | 4.5 | 4.5 |
| 10 | 10 | 218 | ± | 8.3 | 3.8 |

TABLE 3

| Comparative Example No. | Number of Assay Repetitions | hCG (mIU/ml) | | | Variation Coefficient (%) |
|---|---|---|---|---|---|
| | | Average Value | ± | Standard Deviation | |
| 2 | 10 | 25 | ± | 5.3 | 21.2 |
| 2 | 10 | 103 | ± | 16.3 | 15.8 |
| 2 | 10 | 241 | ± | 31.6 | 13.1 |
| 5 | 10 | 25 | ± | 5.3 | 21.2 |
| 5 | 10 | 103 | ± | 16.3 | 15.8 |
| 5 | 10 | 241 | ± | 31.6 | 13.1 |

## TABLE 4

| Example No. | Number of Assay Repetitions | hCG (mIU/ml) Average ± Standard Value Deviation | | Variation Coefficient (%) |
|---|---|---|---|---|
| 2 | 6 | 51 | ± 4.2 | 8.2 |
| 2 | 6 | 185 | ± 12.4 | 6.7 |
| 6 | 6 | 48 | ± 3.6 | 7.5 |
| 6 | 6 | 181 | ± 15.2 | 8.4 |
| 9 | 6 | 51 | ± 4.2 | 8.2 |
| 9 | 6 | 185 | ± 12.4 | 6.7 |
| 10 | 6 | 45 | ± 2.9 | 6.4 |
| 10 | 6 | 183 | ± 9.2 | 5.0 |

## TABLE 5

| Comparative Example No. | Number of Assay Repetitions | hCG (mIU/ml) Average ± Standard Value Deviation | | Variation Coefficient (%) |
|---|---|---|---|---|
| 2 | 6 | 53 | ± 5.8 | 16.9 |
| 2 | 6 | 195 | ± 22.4 | 11.5 |
| 5 | 6 | 53 | ± 5.8 | 16.9 |
| 5 | 6 | 195 | ± 22.4 | 11.5 |

It can he seen from these results that smaller variations in the results of the sandwich enzyme immunoassay can be obtained when at least one of the solid-phase and labeled antibodies is originated from guinea pig than when neither the solid-phase antibody nor the labeled antibody is originated from guinea pig.

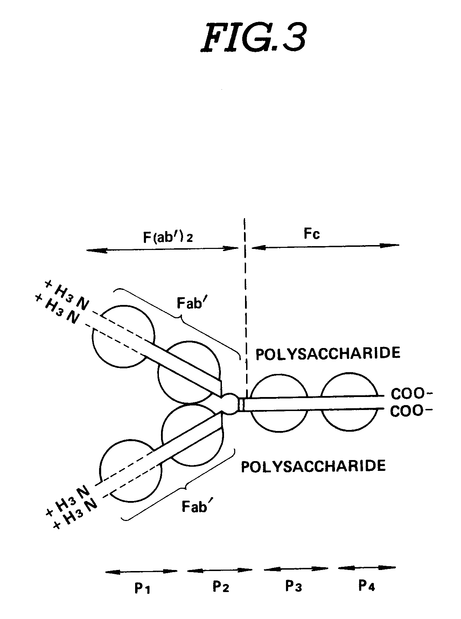

Referring to Fig. 3, there is illustrated in a schematic form the structure of Immunoglobulin G (IgG). The IgG structure can be divided into an antigen-binding $F(ab')_2$ fragment and a crystallizable (Fc) fragment when it is held at a temperature of 37°C for 18 hours in the presence of pepsin. The Fc fragment has a portion P4 for adsorption to a cell. The Fc fragment has no ability of binding to antigen and is crystallized at low temperature. The F(ab')$_2$ fragment can be divided under a reduction process, for example, in the presence of mercaptoethylamine, into two Fab' fragments having a portion P1 capable of binding to antigen. The inventors found that the degree of non-specific binding of the labeled antibody to the solid phase is dependent on the presence of the Fc fragment and can be reduced by replacing the IgG-GOD with Fab'-GOD when at least one of the solid-phase and labeled antibodies is originated from guinea pig. The Fab' fragment is prepared as the result of separation of the Fc fragment from IgG in the following manner:

First of all, 2 ml saturated ammonium sulfate is dripped into 2 ml of the serum drawn from a selected animal as described previously. The resulting solution is agitated slowly at a temperature of 4°C for two or three hours. The resulting cloudy solution is then placed in a centrifugation tube. Centrifugation is performed at a temperature of 4°C with a contrifugal separator rotating at a speed of 3000 rpm for 15 minutes. The supernatuant is removed from the centrifugation tube, whereas the precipitate is dissolved with 2 ml of 0.1 mol/l sodium phosphate buffer (pH 6.0) containing 5 mmol/l ethylenediaminetetraacetic acid (EDTA). The resulting solution is eluted at a flow rate of 6 ml/hour on a Sephacryl S-300 (a trademark of Pharmacia Fine Cehmicals, Sweden) column (1 x 90 cm) equilibrated with 0.1 mol/l acetate buffer (pH 4.5) containing 0.1 mmol/l NaCl. The eluted solution is divided into 1 ml of samples. The IgG fractions, which absorb light at 280 nm, are gathered from the respective samples. Following this, 0.1 ml of 3 mg/ml pepsin solution is added to the gathered IgG. The resulting solution is incubated at a temperature of 37°Cfor 18 hours. The resulting solution is eluted at a flow rate of 6 ml/hour on a Sephacryl S-200 (a trademark of Pharmacia Fine Chemicals, Sweden) column (1 x 90 cm) equilibrated with 0.1 mol/l sodium phosphate buffer (pH 7.5). The eluted solution is divided into 1 ml of samples. The F(ab')2 fractions, which absorb light at 280 nm, are gathered from the respective samples. The gathered F(ab')2 is added with 0.05 ml of 0.1 mol/l mercaptoethylamine and incubated at a temperature of 37°Cfor 90 minutes. The resulting solution is desalted at a flow rate of 6 ml/hour on a Sephadex G-25 (a trademark of Pharmacia Fine Chemicals. Sweden) column (1 x 30 cm) equilibrated with 0.1 mol/l sodium phosphate buffer (pH 6.0). The desalted solution is divided into 1 ml of Fab' samples.

A large number of tests were conducted substantially in the same manner as described previously to prove the advantageous effects on the non-specific binding of the Fab'-GOD labeled antibody to the solid phase.

Example 13 - The labeled antibody used was guinea pig anti-AFP Fab'-GOD and the solid-phase antibody used was guinea pig anti-AFP IgG. A guinea pig anti-AFP IgG-coated polystyrene ball was incubated with the guinea pig anti-AFP Fab'-GOD in the presence of the reaction solution A mixed with guinea pig serum.

Example 14 - The labeled antibody used was guinea pig anti-AFP Fab'-GOD and the solid-phase antibody used was rabbit anti-AFP IgG. A rabbit anti-AFP IgG-coated polystyrene ball was incubated with the guinea pig anti-AFP Fab'-GOD in the presence of the reaction solution A mixed with guinea pig serum.

Example 15 - The labeled antibody used was guinea pig anti-AFP Fab'-GOD and the solid-phase antibody used was goat anti-AFP IgG. A goat anti-AFP IgG-coated polystyrene ball was incubated with the guinea pig anti-AFP Fab'-GOD in the presence of the reaction solution A mixed with guinea pig serum.

Example 16 - The labeled antibody used was rabbit anti-AFP Fab'-GOD and the solid-phase antibody used was guinea pig anti-AFP IgG. A guinea pig anti-AFP IgG-coated polystyrene ball was incubated with the rabbit anti-AFP Fab'-GOD in the presence of the reaction solution A mixed with guinea pig serum.

Example 17 - The labeled antibody used was goat anti-AFP Fab'-GOD and the solid-phase antibody used was guinea pig anti-AFP IgG. A guinea pig anti-AFP IgG-coated polystyrene ball was incubated with the goat anti-AFP Fab'-GOD in the presence of the reaction solution A mixed with guinea pig serum.

Example 18 - The labeled antibody used was guinea pig anti-CEA Fab'-GOD and the solid-phase antibody used was guinea pig anti-CEA IgG. A guinea pig anti-CEA IgG-coated polystyrene ball was incubated with the guinea pig anti-CEA Feb'-GOD in the presence of the reaction solution A mixed with guinea pig serum.

Example 19 - The labeled antibody used was guinea pig anti-CEA Fab'-GOD and the solid-phase antibody used was rabbit anti-CEA IgG. A rabbit anti-CEA IgG-coated polystyrene ball was incubated with the guinea pig anti-CEA Fab'-GOD in the presence of the reaction solution A mixed with guinea pig serum.

Example. 20 - The labeled antibody used was guinea pig anti-CEA Fab'-GOD and the solid-phase antibody used was goat anti-CEA IgG. A goat anti-CEA IgG-coated polystyrene ball was incubated with the guinea pig anti-CEA Fab'-GOD in the presence of the reaction solution A mixed with guinea pig serum.

Example 21 - The labeled antibody used was rabbit anti-CEA Fab'-GOD and the solid-phase antibody used was guinea pig anti-CEA IgG. A guinea pig anti-CEA IgG-coated polystyrene ball was incubated with the rabbit anti-CEA Fab'-GOD in the presence of the reaction solution A mixed with guinea pig serum.

Example 22 - The labeled antibody used was goat anti-CEA Fab'-GOD and the solid-phase antibody used was guinea pig anti-CEA IgG. A guinea pig anti-CEA IgG-coated polystyrene ball was incubated with the goat anti-CEA Feb'-GOD in the presence of the reaction solution A mixed with guinea pig serum.

For comparison of the non-specific binding reduction effect obtainable by the invention, tests were conducted for the following comparative examples:

comparative Example 8 - The labeled antibody used was guinea pig anti-AFP IgG-GOD and the solid-phase antibody used was guinea pig anti-AFP IgG. A guinea pig anti-AFP IgG-coated polystyrene ball was incubated with the guinea pig anti-AFP IgG-GOD in the presence of the reaction solution A mixed with guinea pig serum.

Comparative Example 9 - The labeled antibody used was rabbit anti-AFP Fab'-GOD and the solid-phase antibody used was rabbit anti-AFP IgG. A rabbit anti-AFP IgG-coated polystyrene ball was incubated with the rabbit anti-AFP Fab'-GOD in the presence of the reaction solution A mixed with guinea pig serum.

Comparative Example 10 - The labeled antibody used was goat anti-AFP Fab'-GOD and the solid-phase antibody used was goat anti-AFP IgG. A goat anti-AFP IgG-coated polystyrene ball was incubated with the goat anti-AFP Fab'-GOD in the presence of a reaction solution.

Comparative Example 11 - The labeled antibody used was guinea pig anti-CEA IgG-GOD and the solid-phase antibody used was guinea pig anti-CEA IgG. A guinea pig anti-CEA IgG-coated polystyrene ball was incubated with the guinea pig anti-CEA IgG-GOD in the presence of the reaction solution A mixed with guinea pig serum.

Comparative Example 12. The labeled antibody used was rabbit anti-CEA Fab'-GOD and the solid-phase antibody used was rabbit anti-CEA IgG. A rabbit anti-CEA IgG-coated polystyrene ball was incubated with the rabbit anti-CEA Fab'-GOD in the presence of the reaction solution A mixed with guinea pig serum.

Comparative Example 13 - The labeled antibody used was goat anti-CEA Fab'-GOD and the solid-phase antibody used was goat anti-CEA IgG. A goat anti-CEA IgG-coated polystyrene ball was incubated with the goat anti-CEA Fab'-GOD in the presence of the reaction solution A mixed with guinea pig serum.

The results of the non-specific binding tests are illustrated in Table 6.

TABLE 6

| Example No. | Average Non-Specific Binding Percentage (%) | Comparative Example No. | Average Non-Specific Binding Percentage (%) |
|---|---|---|---|
| 13 | $4.3 \times 10^{-3}$ | 8 | $1.3 \times 10^{-2}$ |
| 14 | $4.5 \times 10^{-3}$ | 9 | $2.3 \times 10^{-1}$ |
| 15 | $3.9 \times 10^{-3}$ | 10 | $1.2 \times 10^{-2}$ |
| 16 | $2.8 \times 10^{-3}$ | 11 | $1.5 \times 10^{-2}$ |
| 17 | $4.7 \times 10^{-3}$ | 12 | $1.4 \times 10^{-2}$ |
| 18 | $3.6 \times 10^{-3}$ | 13 | $1.8 \times 10^{-2}$ |
| 19 | $3.9 \times 10^{-3}$ | | |
| 20 | $4.4 \times 10^{-3}$ | | |
| 21 | $4.1 \times 10^{-3}$ | | |
| 22 | $3.8 \times 10^{-3}$ | | |

The non-specific binding percentages of the Comparative Examples 9, 10, 12 and 13 where the labeled antibody was Fab'-GOD are substantially the same as those of the Comparative Examples 8 and 11 where both of the IgG solid-phase antibody and the IgG-GOD labeled antibody were originated from guinea pig. It can be seen from the test results related to the Examples 13-22 that the degree of non-specific binding of the labeled antibody to the solid phase can be reduced to a remarkable extent when at least one of the Fab'-GOD labeled antibody and the IgG solid-phase antibody are originated from guinea pig.

The measurable range and detection limit of sandwich enzyme immunoassay were determined substantially in the same manner as described previously. The results of the sandwich enzyme immunoassay measurable range and detection limit are illustrated in Tables 7-10.

13

TABLE 7

| Example No. | Measurable Range AFP (ng/ml) | Comparative Example No. | Measurable Range AFP(ng/ml) |
|---|---|---|---|
| 13 | 0.1 50 | 8 | 1.0 - 100 |
| 14 | 0.1 - 50 | 9 | 2.5 - 100 |
| 15 | 0.1 - 50 | 10 | 1.0 - 100 |
| 16 | 0.1 - 50 | | |
| 17 | 0.1 - 50 | | |

TABLE 8

| Example No. | Measurable Range CEA (ng/ml) | Comparative Example No. | Measurable Range CEA (ng/ml) |
|---|---|---|---|
| 18 | 0.1 - 100 | 11 | 2.5 - 250 |
| 19 | 0.1 - 100 | 12 | 1.0 - 250 |
| 20 | 0.1 - 100 | 13 | 5.0 - 250 |
| 21 | 0.1 - 100 | | |
| 22 | 0.1 - 100 | | |

TABLE 9

| Example No. | Detection Limit AFP (ng/tube) | Comparative Example No. | Detection Limit AFP (ng/tube) |
|---|---|---|---|
| 13 | 0.01 | 8 | 0.1 |
| 14 | 0.01 | 9 | 0.25 |
| 15 | 0.01 | 10 | 0.1 |
| 16 | 0.01 | | |
| 17 | 0.01 | | |

TABLE 10

| Example No. | Detection Limit CEA (ng/tube) | Comparative Example No. | Detection Limit CEA (ng/tube) |
|---|---|---|---|
| 18 | 0.01 | 11 | 0.25 |
| 19 | 0.01 | 12 | 0.1 |
| 20 | 0.01 | 13 | 0.5 |
| 21 | 0.01 | | |
| 22 | 0.01 | | |

It can be seen from these test results that the measurable range and detection limit of sandwich enzyme immunoassay are superior when the labeled antibody used is Fab'-GOD to those obtained when the labeled antibody used is IgG-GOD. In addition, the measurable range and detection limit of sandwich enzyme immunoassay are superior when at least one of the labeled antibody (Fab'-GOD) and the solid-phase antibody (IgG) is originated from guinea pig to those obtained when the labeled antibody is Fab'-GOD originated from rabbit and the solid-phase antibody (IgG) is originated from rabbit or when the labeled antibody (Fab'-GOD) is originated from goat and the solid-phase antibody (IgG) is originated from goat.

Sandwich enzyme immunoassay was repeated ten times within a day (within assay) using the antibodies in Examples 13-22 and Comparative Examples 8-13. The results are shown in Tables 11-14.

14

Similarly, sandwich enzyme immunoassay was repeated six times on different six days (between assay) using the antibodies in Examples 13-22 and Comparative Examples 8-13. The results are shown in Tables 15-18.

## TABLE 11

| Example No. | Number of Assay Repetitions | AFP (ng/ml) | | Variation Coefficient (%) |
|---|---|---|---|---|
| | | Average Value ± | Standard Deviation | |
| 13 | 10 | 2.4 ± | 0.079 | 3.3 |
| 14 | 10 | 2.7 ± | 0.13 | 4.8 |
| 15 | 10 | 2.5 ± | 0.075 | 3.0 |
| 16 | 10 | 2.6 ± | 0.073 | 2.8 |
| 17 | 10 | 2.5 ± | 0.090 | 3.6 |

## TABLE 12

| Comparative Example No. | Number of Assay Repetitions | AFP (ng/ml) | | Variation Coefficient (%) |
|---|---|---|---|---|
| | | Average Value ± | Standard Deviation | |
| 8 | 10 | 2.5 ± | 0.13 | 5.0 |
| 9 | 10 | 2.9 ± | 0.36 | 12.5 |
| 10 | 10 | 2.8 ± | 0.39 | 13.9 |

TABLE 13

| Example No. | Number of Assay Repetitions | CEA (ng/ml) Average ± Standard Value Deviation | | | Variation Coefficient (%) |
|---|---|---|---|---|---|
| 18 | 10 | 5.2 | ± | 0.19 | 3.6 |
| 19 | 10 | 5.3 | ± | 0.27 | 5.1 |
| 20 | 10 | 4.8 | ± | 0.24 | 5.0 |
| 21 | 10 | 5.5 | ± | 0.19 | 3.4 |
| 22 | 10 | 5.2 | ± | 0.12 | 2.3 |

TABLE 14

| Comparative Example No. | Number of Assay Repetitions | CEA (ng/ml) Average ± Standard Value Deviation | | | Variation Coefficient (%) |
|---|---|---|---|---|---|
| 11 | 10 | 5.1 | ± | 0.20 | 3.9 |
| 12 | 10 | 5.2 | ± | 0.93 | 18.2 |
| 13 | 10 | 5.5 | ± | 0.91 | 16.5 |

16

TABLE 15

| Example No. | Number of Assay Repetitions | AFP (ng/ml) Average ± Standard Value Deviation | | Variation Coefficient (%) |
|---|---|---|---|---|
| 13 | 6 | 2.6 | ± 0.091 | 3.5 |
| 14 | 6 | 2.3 | ± 0.13 | 5.6 |
| 15 | 6 | 2.4 | ± 0.15 | 6.2 |
| 16 | 6 | 2.3 | ± 0.10 | 4.5 |
| 17 | 6 | 2.6 | ± 0.099 | 3.8 |

TABLE 16

| Comparative Example No. | Number of Assay Repetitions | AFP (ng/ml) Average ± Standard Value Deviation | | Variation Coefficient (%) |
|---|---|---|---|---|
| 8 | 6 | 2.3 | ± 0.090 | 3.9 |
| 9 | 6 | 2.4 | ± 0.38 | 15.8 |
| 10 | 6 | 2.6 | ± 0.48 | 18.6 |

## TABLE 17

| Example No. | Number of Assay Repetitions | CEA (ng/ml) Average ± Standard Value Deviation | | Variation Coefficient (%) |
|---|---|---|---|---|
| 18 | 6 | 5.1 | ± 0.26 | 5.0 |
| 19 | 6 | 5.0 | ± 0.24 | 4.8 |
| 20 | 6 | 5.2 | ± 0.19 | 3.6 |
| 21 | 6 | 5.3 | ± 0.25 | 4.7 |
| 22 | 6 | 5.0 | ± 0.26 | 5.1 |

## TABLE 18

| Comparative Example No. | Number of Assay Repetitions | CEA (ng/ml) Average ± Standard Value Deviation | | Variation Coefficient (%) |
|---|---|---|---|---|
| 11 | 6 | 5.3 | ± 0.19 | 3.6 |
| 12 | 6 | 5.3 | ± 0.83 | 15.6 |
| 13 | 6 | 5.3 | ± 0.78 | 14.8 |

It can be seen from these results that smaller variations in the results of the sandwich enzyme immunoassay can be obtained when at least one of the labeled antibody (Fab'-GOD) and the solid-phase antibody (IgG) is originated from guinea pig.

The inventors also found that the degree of non-specific binding of the labeled antibody to the solid phase can be reduced by adding normal guinea pig serum to the reaction solution A in the present of which the labeled antibody binds to the solid-phase antibody through the antigen. A number of tests were conducted to provide the advantageous effect on the degree of non-specific binding of the labeled antibody to the solid phase.

Example 23 - The labeled antibody used was guinea pig anti-hCG IgG-GOD and the solid-phase antibody used was guinea pig anti-hCG IgG. The reaction solution was a mixture of the reaction solution A and normal guinea pig serum.

The degree of non-specific binding of the labeled antibody to the solid phase was determined. For thin purpose, a guinea pig anti-hCG IgG-coated polystyrene ball was incubated at room temperature with guinea pig anti-hCG IgG diluted with 0.3 ml of a reaction solution mixture X containing the reaction solution A (0.056 mol/l sodium phosphate buffer (pH 6.3) containing 0.1% sodium azide, 0.2% bovine serum albumin (BSA) and 0.337% NaCl) mixed at a volume ratio with normal guinea pig serum in an assay tube all the night through. The polystyrene ball was washed three times with the reaction solution A and then transferred into another clean assay tube. Following this, 0.3 ml of 0.01 mol/l acetate buffer (pH 5.1) containing 0.5 mol/l glucose was added to the assay tube. The assay tube was held stationary at a temperature of 37°C for two hours. The resulting solution was taken to prepare a 0.1 ml sample. The

18

sample was added with 0.5 ml of 0.2 mol/l carbonate buffer (pH 9.8) containing $2 \times 10^{-7}$ mol/l luminol and then with 0.5 ml of $6 \times 10^{-3}$ mol/l potassium ferricyanide. The light emitted upon chemiluminenscent reaction of the luminol and potassium ferricyanide the resulting hydrogen peroxide was measured by a Luminometer UPD-8000 (a trademark of Meidensha Electric Mfg., Co., Japan) which starts its counting operation 15 second after the luminol and potassium ferricyanide were added to the assay tube and counted the light emitted for 30 seconds. The degree of non-specific binding of the labeled antibody to the solid phase was represented by the non-specific binding ratio given as C1/C2 x 100 (%) where C1 is the number of counts measured for the labeled antibody binding to the polystyrene ball and C2 is the number of counts corresponding to the whole amount of the labeled antibody used in the assay tube. These procedure were repeated while changing the volume ratio of the normal guinea pig serum to the reaction solution A from 1:0 to 1:20.

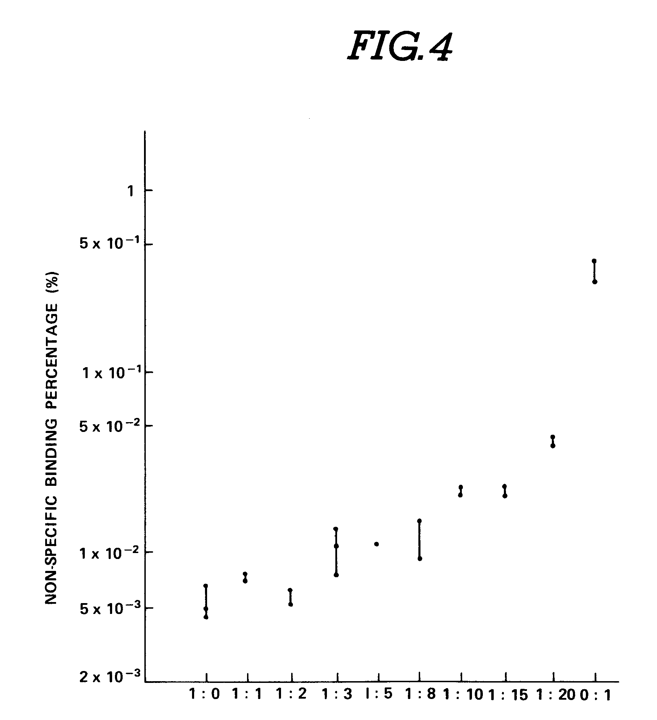

Fig. 4 illustrates the results of a series of non-specific binding tests conducted for different volume ratios. It can be seen from the test results that the non-specific binding percentage increases from 0.005% to 0.05% as the volume ratio of the normal guinea pig serum to the reaction solution A increases from 1:0 to 1:20.

For comparison of the non-specific binding reduction obtainable by the invention, a test was conducted substantially in the same manner for the following comparative example:

Comparative Example 14 - The labeled antibody used was guinea pig anti-hCG IgG-GOD and the solid-phase antibody used was guinea pig anti-hCG IgG. The reaction solution used was the reaction solution A mixed with no normal guinea pig serum. For this comparative example, the non-specific binding ratio was 0.4%.

It is, therefore, apparent that the degree of non-specific binding of the labeled antibody to the solid phase can be reduced to a considerable extent when normal guinea pig serum is added to the reaction solution used in binding the labeled antibody to the solid-phase antibody.

The sensitivity and measurable range of sandwich enzyme immunoassay was determined for the following example:

Example 24 - The labeled antibody used was guinea pig anti-hCG IgG-GOD and the solid-phase antibody used was guinea pig anti-hCG IgG. The reaction solution used was a mixture of the raction solution A and normal guinea pig serum. The volume ratio of the normal guinea pig serum to the reaction solution A was 1:5.

An anti-hCG IgG-coated polystyrene ball was incubated at room temperature with hCG standard solution diluted with 0.3 ml of reaction solution A in an assay tube for six hours. The polystyrene ball was washed three times with the reaction solution A. The washed polystyrene ball was incubated with 0.1 ml of anti-hCG IgG-GOD labeled antibody diluted at a proper ratio with a mixture of the reaction solution A mixed with normal guinea pig serum. The volume ratio of the normal guinea pig serum to the reaction solution A was 1:5. The assay tube was held stationary at room temperature all the night through. The polystyrene ball was then washed three times with the reaction solution A and transferred into another clean assay tube. Following this. 0.3 ml of 0.01 mol/l acetate buffer (pH 5.1) containing 0.5 mol/l glucose was added to the assay tube. The assay tube was held stationary at a temperature of 37°C for two hours. The resulting solution was taken to prepare a 0.1 ml sample. The sample was added with 0.5 ml of 0.2 mol/l carbonate buffer (pH 9.8) containing $2 \times 10^{-7}$ mol/l luminol and then with 0.5 ml of $6 \times 10^{-3}$ mol/l potassium ferricyanide. The light emitted upon chemiluminescent reaction of the luminol and potassium ferricyanide and the resulting hydrogen peroxide was measured by a Luminometer UPD-8000 which started its operation 15 seconds after the luminol and potassium ferricyanide were added to the assay tube and counted the light emitted for 30 seconds.

Similar procedures were repeated for the following comparative example in order to prove the advantageous effects on the sandwich enzyme immunoassay;

Comparative Example 15 - The labeled antibody used was guinea pig anti-hCG IgG-GOD and the solid-phase antibody used was guinea pig anti-hCG IgG. The reaction solution used was the reaction solution A mixed with no normal guinea pig serum.

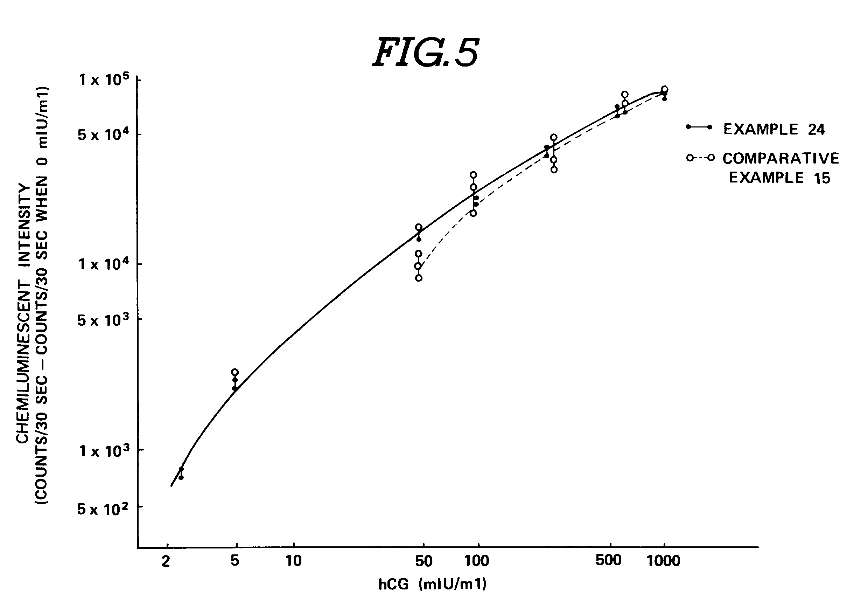

Fig. 5 illustrates the results. The sandwich enzyme immunoassay measurable range and sensitivity obtained for Example 24 were 2.5-1000 mIU/ml and 2.5 mIU/ml, respectively. On the other hand, the sandwich enzyme immunoassay measurable range and sensitivity were 50-500 mIU/ml and 50 mIU/ml, respectively, for Comparative Example 15. It is, therefore, apparent that the sandwich enzyme immunoassay sensitivity and measurable range are improved when normal guinea pig serum is added to the reaction solution A.

Sandwich enzyme immunoassay was repeated ten times within a day (within assay) for Example 24 and Comparative Example 15. The results are shown in Tables 19 and 20. Similarly, sandwich enzyme

19

immunoassay was repeated on different days (between assay) for Example 24 and Comparative Example 15. The results are shown in Tabels 21 and 22.

TABLE 19

| Example No. | Number of Assay Repetitions | hCG (mIU/ml) | | Variation Coefficient (%) |
|---|---|---|---|---|
| | | Average Value ± | Standard Deviation | |
| 24 | 10 | 13.5 ± | 1.1 | 7.8 |
| 24 | 10 | 124.3 ± | 6.6 | 5.3 |
| 24 | 10 | 207.5 ± | 10.0 | 4.8 |

TABLE 20

| Comparative Example No. | Number of Assay Repetitions | hCG (mIU/ml) | | Variation Coefficient (%) |
|---|---|---|---|---|
| | | Average Value ± | Standard Deviation | |
| 15 | 10 | 52.3 ± | 13.2 | 25.3 |
| 15 | 10 | 230.0 ± | 35.9 | 15.6 |
| 15 | 10 | 450.0 ± | 87.8 | 19.5 |

TABLE 21

| Example No. | Number of Assay Repetitions | hCG (mIU/ml) | | Variation Coefficient (%) |
|---|---|---|---|---|
| | | Average Value ± | Standard Deviation | |
| 24 | 6 | 57.3 ± | 3.7 | 6.5 |
| 24 | 6 | 180.1 ± | 6.8 | 3.8 |

TABLE 22

| Comparative Example No. | Number of Assay Repetitions | hCG (mIU/ml) | | Variation Coefficient (%) |
|---|---|---|---|---|
| | | Average Value | ± Standard Deviation | |
| 15 | 6 | 85.2 | ± 11.2 | 13.2 |
| 15 | 6 | 251.5 | ± 27.4 | 10.9 |

It can be seen from these results that smaller variations in the results of the sandwich enzyme immunoassay can be obtained when normal guinea pig serum is added to the reaction solution.

In order to provide the effective serum to be added to the reaction solution, a number of tests were conducted for the following comparative examples:

Comparative Example 16 - The labeled antibody used was guinea pig anti-hCG IgG-GOD and the solid-phase antibody used was guinea pig anti-hCG IgG. Normal rabbit serum was added to the reaction solution A. The volume ratio of the normal rabit serum to the reaction solution A was 1:5.

Comparative Example 17 - The labeled antibody used was guinea pig anti-hCG IgG-GOD and the solid-phase antibody used was guinea pig anti-hCG IgG. Normal goat serum was added to the reaction solution A. The volume ratio of the normal goat serum to the reaction solution A was 1:5.

Comparative Example 18 - The labeled antibody used was guinea pig anti-hCG IgG-GOD and the solid-phase antibody used was guinea pig anti-hCG IgG. Normal horse serum was added to the reaction solution A. The volume ratio of the normal horse serum to the reaction solution A was 1:5.

Comparative Example 19 - The labeled antibody used was guinea pig anti-hCG IgG-GOD and the solid-phase antibody used was guinea pig anti-hCG IgG. No serum was added to the reaction solution A.

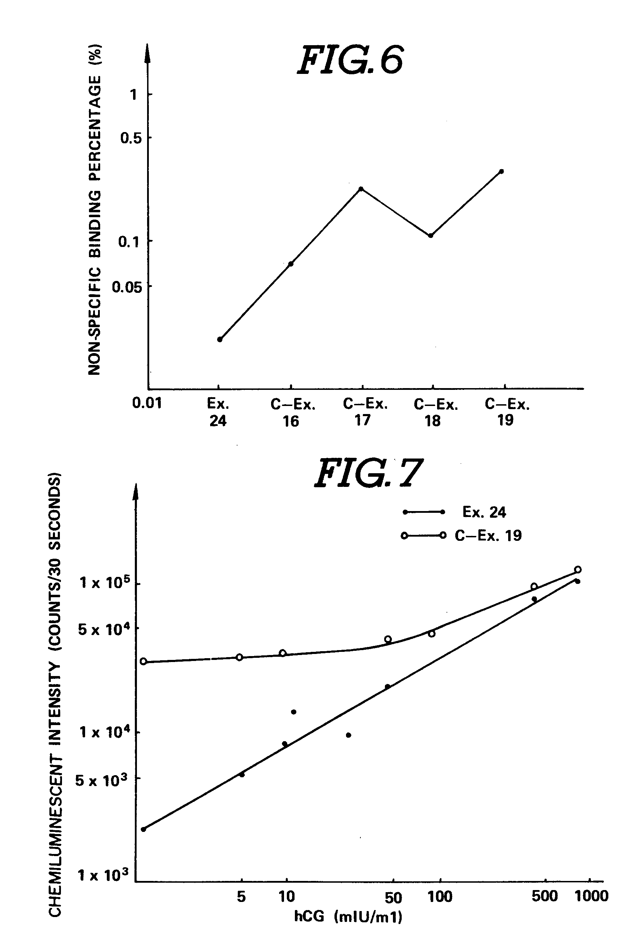

The results of the non-specific binding tests conducted substantially in the same manner as described previously are illustrated In Fig. 6. The non-specific binding percentage was as small as 0.022% for Example 24. On the other hand, for Comparative Examples 16-19, the non-specific binding percentage ranged from 0.07% to 0.3%. It is, therefore, apparent that the degree of non-specific binding of the labeled antibody to the solid phase can be reduced to a remarkable extent by adding normal guinea pig serum to the, reaction solution.

The results of the sandwich enzyme immunoassay sensitivity and sensitive range tests conducted substantially in the same manner as described previously are illustrated in Fig. 7. The sandwich enzyme immunoassay sensitive range and sensitivity were 1-1000 mIU/ml and 1 mU/ml for Example 24. On the other hand, for Comparative Example 19, the sandwich enzyme immunoassay sensitive range and sensitivity were 50-1000 mIU/ml and 50 mIU/ml. It is, therefore, apparent that the sandwich enzyme immunoassay sensitive range and sensitivity can be improved by adding normal guinea pig serum to the reaction solution.

Sandwich enzyme immunoassay was repeated ten times within a day (within assay) for Example 24 and Comparative Example 19. The results are shown in Tables 23 and 24. Similarly, sandwich enzyme immunoassay was repeated on different days (between assay) for Example 24 and Comparative Example 19. The results are shown in Tables 25 and 26.

## TABLE 23

| Example No. | Number of Assay Repetitions | hCG (mIU/ml) Average ± Standard Value Deviation | | Variation Coefficient (%) |
|---|---|---|---|---|
| 24 | 10 | 33 | ± 2.9 | 8.9 |
| 24 | 10 | 108 | ± 5.4 | 5.0 |
| 24 | 10 | 520 | ± 18.7 | 3.6 |

## TABLE 24

| Comparative Example No. | Number of Assay Repetitions | hCG (mIU/ml) Average Value | ± | Standard Deviation | Variation Coefficient (%) |
|---|---|---|---|---|---|
| 19 | 10 | 43 | ± | 6.8 | 15.8 |
| 19 | 10 | 215 | ± | 30.1 | 14.0 |
| 19 | 10 | 488 | ± | 55.1 | 11.3 |

## TABLE 25

| Example No. | Number of Assay Repetitions | hCG (mIU/ml) Average Value | ± | Standard Deviation | Variation Coefficient (%) |
|---|---|---|---|---|---|
| 24 | 6 | 80 | ± | 6.2 | 7.7 |
| 24 | 6 | 460 | ± | 18.4 | 4.0 |

## TABLE 26

| Comparative Example No. | Number of Assay Repetitions | hCG (mIU/ml) Average Value | ± | Standard Deviation | Variation Coefficient (%) |
|---|---|---|---|---|---|
| 19 | 6 | 76 | ± | 14.6 | 19.2 |
| 19 | 6 | 510 | ± | 68.3 | 13.4 |

It can be seen from these results that smaller variations in the results of the sandwich enzyme immunoassay can be obtained when normal guinea pig serum is added to the reaction solution.

Although the reaction solution has been described as containing sodium phosphate buffer (pH 6.3), it is to be noted that it may be replaced with sodium phosphate buffer (pH 6.0-7.5), borate buffer (pH 8.0-8.5) or the like. Although the labeled antibody has been describing as containing glucose oxidase (GOD) label enzyme, it is to be noted that the label enzyme may be peroxidase, alkaline phosphatase, $\beta$-D-galactosidase, or the like. In addition, although the invention has been described in connection with sandwich enzyme immunoassay, it is to be noted that the label may be a fluorescent material (fluorescein isothiocyanate, tetramethylrhodamine isothiocyanate, or the like), a chemiluminescent material (aminoethylethyl isoluminol, aminobutylethyl isoluminol, aminopentylethyl isoluminol, aminohexylethyl isoluminol, or the like), or a radioactive isotope. ($^3$H, $^{14}$C, $^{92}$P, $^{125}$I, $^{131}$I, or the like).

**Claims**

1. A kit for use in a sandwich immunoassay employing labeled and solid-phase antibodies reactive with an antigen in the presence of a reaction solution to bind the antigen between the labeled and solid-phase antibodies, said kit containing a solid-phase antibody having the antibody bound to a solid phase, a labeled antibody having a label enzyme bound to the antibody, at least one of the labeled and solid-phase antibodies originating from a guinea pig, and a reaction solution, characterised in that the reaction solution contains normal guinea pig serum for reducing the degree of non-specific binding of the labeled antibody to the solid phase.

2. A kit as claimed in claim 1, wherein the labeled antibody includes immunoglobulin G.

3. A kit as claimed in claim 2, wherein the labeled antibody includes a Fab'fragment produced from the immunoglobulin G.

4. A kit as claimed in claim 1, wherein the labeled antibody is originated from a guinea pig.

5. A kit as claimed in claim 1, wherein the solid-phase antibody originates from a guinea pig.

6. A kit as claimed in claim 1, wherein the labeled antibody originates from a guinea pig, and the solid-phase antibody originates from a guinea pig.

**Patentansprüche**

1. Kit zur Verwendung in einem Sandwich-Immunoassay, das markierte und Festphasen-Antikörper verwendet, die mit einem Antigen in Gegenwart einer Reaktionslösung reaktiv sind, um das Antigen zwischen dem markierten Antikörper und dem Fstphasen-Antikörper zu binden, wobei das Kit einen Festphasen-Antikörper, bei dem der Antikörper an eine feste Phase gebunden ist, einen markierten Antikörper, bei dem ein Markierungsenzym an den Antikörper gebunden ist, wobei wenigstens einer des markierten Antikörpers und des Festphasen-Antikörpers von einem Meerschweinchen stammt, und eine Reaktionslösung enthält, dadurch gekennzeichnet, daß die Reaktionslösung normales Meerschweinchenserum enthält, um das Ausmaß des nicht-spezifischen Bindens des markierten Antikörpers an die feste Phase zu erniedrigen.

2. Kit nach Anspruch 1, in dem der markierte Antikörper Immunglobulin G umfaßt.

3. Kit nach Anspruch 2, in dem der markierte Antikörper ein Fab' Fragment umfaßt, das aus Immunglobulin G hergestellt ist.

4. Kit nach Anspruch 1, in dem der markierte Antikörper von einem Meerschweinchen stammt.

5. Kit nach Anspruch 1, in dem der Festphasen-Antikörper von einem Meerschweinchen stammt.

6. Kit nach Anspruch 1, in dem der markierte Antikörper von einem Meerschweinchen stammt, und der Festphasen-Antikörper von einem Meerschweinchen stammt.

**Revendications**

1. Trousse pour utilisation dans un immuno-essai sandwich, utilisant des anticorps marqués et en phase solide qui réagissent avec un antigène en présence d'une solution de réaction pour lier l'antigène entre les anticorps marqués et en phase solide, ladite trousse contenant un anticorps en phase solide où l'anticorps est lié à une phase solide, un anticorps marqué où un enzyme de marquage est lié à l'anticorps, l'un au moins des anticorps marqué et en phase solide provenant d'un cobaye, et une solution de réaction, caractérisée en ce que la solution de réaction contient du sérum normal de cobaye pour réduire le degré de liaison non spécifique de l'anticorps marqué à la phase solide.

2. Trousse selon la revendication 1, caractérisée en ce que l'anticorps marqué comprend de l'immunoglobuline G.

3. Trousse selon la revendication 2, caractérisée en ce que l'anticorps marqué comprend un fragment Fab produit à partir de l'immunoglobuline G.

4. Trousse selon la revendication 1, caractérisée en ce que l'anticorps marqué provient d'un cobaye.

5. Trousse selon la revendication 1, caractérisée en ce que l'anticorps en phase solide provient d'un cobaye.

6. Trousse selon la revendication 1, caractérisée en ce que l'anticorps marqué provient d'un cobaye, et l'anticorps en phase solide provient d'un cobaye.

# FIG.1

EP 0 249 955 B1

# FIG.2

○ EXAMPLE 3
△ EXAMPLES 7, 11
□ EXAMPLE 8
◇ EXAMPLE 12
● COMPARATIVE EXAMPLE 3
▲ COMPARATIVE EXAMPLE 7
■ COMPARATIVE EXAMPLE 6

CHEMILUMINESCENT INTENSITY (COUNTS/30 SECONDS)

AFP (ng/m1)

# FIG.3

# FIG.4

FIG.5

EP 0 249 955 B1

FIG.6

FIG.7